# EUROPÄISCHE PATENTANMELDUNG

(11) **EP 4 802 890 A1**
(43) Veröffentlichungstag der Anmeldung: **09.09.2026**
(21) Anmeldenummer: 26162194.0
(22) Anmeldetag: 04.03.2026
(51) Int. Cl.: A01N 1/14, A01N 1/144, C12N 5/00, F25D 25/00

(54) **AUTOMATISIERTES BLUTKONSERVENDEPOT UND VERFAHREN ZUR HANDHABUNG VON BLUTKONSERVEN**

(30) Priorität: 05.03.2025 DE 202025101178 U
(71) Anmelder: LabMatic Automation GmbH, 31515 Wunstorf (DE)
(72) Erfinder: Lange, Mario, 31515 Wunstorf (DE)
(74) Vertreter: Werner, André

(57) **Zusammenfassung**

Die Erfindung betrifft ein automatisiertes Blutkonservendepot (100) sowie ein Verfahren zur Handhabung von Blutkonserven (200). Das Blutkonservendepot (100) umfasst eine Schutzeinhausung (110) zur Abschirmung eines Depotbereichs (301), einen Handhabungsroboter (120) zur automatisierten Handhabung der Blutkonserven (200) in einem Handhabungsbereich (302), eine Durchreichvorrichtung (130) mit einem Einlagerungsbereich (303) und einem hiervon getrennten Auslagerungsbereich (304), eine im Depotbereich (301) angeordnete Kühlvorrichtung (140) zur temperierten Lagerung der Blutkonserven (200), eine Etikettenvorrichtung (150) zur Erfassung und/oder Erstellung von Etiketten sowie eine Steuereinrichtung (160) zur Koordination der Komponenten. Der Handhabungsroboter (120) ist dazu eingerichtet, einzulagernde Blutkonserven (200) aus dem Einlagerungsbereich (303) zu entnehmen und in die Kühlvorrichtung (140) einzulagern sowie auszulagernde Blutkonserven (200) aus der Kühlvorrichtung (140) zu entnehmen und im Auslagerungsbereich (304) bereitzustellen. Die Erfindung ermöglicht eine automatisierte, sichere und nachvollziehbare Ein- und Auslagerung von Blutkonserven (200).

## Beschreibung

Die Erfindung betrifft ein automatisiertes Blutkonservendepot und ein Verfahren zur Handhabung von Blutkonserven unter Verwendung eines solchen Blutkonservendepots.

Der Gesundheitssektor ist in zunehmendem Maße mit steigenden Personalkosten und wachsendem wirtschaftlichem Druck konfrontiert, sodass der Einsatz automatisierter Prozesse zur Effizienzsteigerung an Bedeutung gewinnt. Diese Entwicklung betrifft auch Einrichtungen zur Lagerung und Bereitstellung medizinischer Verbrauchsgüter, wie Blutkonservendepots. Blutkonservendepots dienen der Aufbewahrung von Blutkonserven, in denen insbesondere menschliches Blut unter definierten Temperaturbedingungen gelagert wird. Solche Blutkonservendepots können beispielsweise als Blutkonserven-Kühlgeräte ausgebildet sein oder ein solches umfassen und sind zur Lagerung von Blutkonserven bei niedrigen Temperaturen vorgesehen, etwa in einem Temperaturbereich von 2 °C bis 6 °C. Ein derartiger Temperaturbereich ist erforderlich, um die Verwendbarkeit der Blutkonserven, beispielsweise für Bluttransfusionen, sicherzustellen.

Die geordnete Einlagerung von Blutkonserven in ein Blutkonservendepot sowie deren bedarfsgerechte Auslagerung erfordern üblicherweise eine manuelle Handhabung durch Bedienpersonal, etwa durch Laborpersonal. Diese manuelle Handhabung ist zeitintensiv und damit kostenaufwendig. Folglich besteht im Zusammenhang mit der Handhabung von Blutkonserven in Blutkonservendepots ein erhebliches Potenzial zur Kosteneinsparung durch Automatisierung.

Es ist daher eine Aufgabe der vorliegenden Erfindung, eine kostengünstige und effiziente Handhabung von Blutkonserven zu ermöglichen.

Diese Aufgabe wird durch ein automatisiertes Blutkonservendepot mit den Merkmalen nach Anspruch 1 sowie durch ein Verfahren zur Handhabung von Blutkonserven nach Anspruch 15 gelöst. Zweckmäßige Weiterbildungen der Erfindung sind in den Ansprüchen 2 bis 14 angegeben.

Ein erster Aspekt der Erfindung betrifft ein automatisiertes Blutkonservendepot zur Handhabung von Blutkonserven. Die Handhabung der Blutkonserven umfasst sowohl die Einlagerung dem Blutkonservendepot zugeführter Blutkonserven als auch deren bedarfsgerechte Bereitstellung, das heißt die Auslagerung aus dem Blutkonservendepot. Die Blutkonserven sind vorzugsweise flexibel ausgebildet, sodass sie nicht zwingend formstabil sind, beispielsweise wenn sie in einem Blutbeutel aufgenommen sind. Die Blutkonserven können vor ihrer Einlagerung bereits in weitere Behältnisse, beispielsweise Röhrchen, überführt und in einem Labor-Informationssystem dokumentiert sein.

Das Blutkonservendepot umfasst eine Schutzeinhausung, welche einen Depotbereich zumindest teilweise gegenüber der Umgebung abschirmt.

Zudem umfasst das Blutkonservendepot einen Handhabungsroboter, der dazu eingerichtet ist, Blutkonserven in einem Handhabungsbereich zu handhaben, der sich zumindest teilweise innerhalb des Depotbereichs erstreckt. Die Handhabung der Blutkonserven erfolgt, indem der Handhabungsroboter Blutkonserven greift, bewegt und/oder ablegt.

Das Blutkonservendepot umfasst ferner eine Durchreichvorrichtung, die an einer die Schutzeinhausung durchsetzenden Durchreichöffnung an den Depotbereich angeschlossen ist, sodass der Depotbereich über die Durchreichöffnung von außen zugänglich ist. Dabei erstreckt sich der Handhabungsbereich derart über die Durchreichvorrichtung, dass der Handhabungsroboter in die Durchreichvorrichtung hineingreifen kann. Auf diese Weise können Blutkonserven mittels des Handhabungsroboters auch innerhalb der Durchreichvorrichtung gehandhabt werden. Die Schutzeinhausung kann mindestens eine Tür, insbesondere eine Flügeltür, aufweisen, über die ein Zugang zum Depotbereich ermöglicht wird.

Weiterhin umfasst das Blutkonservendepot mindestens eine im Depotbereich angeordnete Kühlvorrichtung zur Kühlung und Lagerung von Blutkonserven. Die Kühlvorrichtung ist zur dauerhaften Temperierung der darin eingelagerten Blutkonserven ausgelegt, wobei die Aufbewahrung beispielsweise in einem Temperaturbereich von 2 °C bis 6 °C erfolgt. Die Kühlvorrichtung kann auch als Blutkonserven-Kühlgerät oder Kühlschrank bezeichnet werden. Vorzugsweise ist die Kühlvorrichtung zur Aufnahme einer Mehrzahl von Blutkonserven ausgebildet, insbesondere zur Aufnahme von mindestens 100, bevorzugt etwa 120 Blutkonserven. Die Blutkonserven können hierzu in definierten Lagerpositionen innerhalb der Kühlvorrichtung angeordnet sein. Die Kühlvorrichtung ist vorzugsweise als medizinisches Kühlgerät ausgebildet und erfüllt insbesondere die Anforderungen der europäischen Medizinprodukteverordnung (EU) 2017/745 (MDR).

Des Weiteren umfasst das Blutkonservendepot eine Etikettenvorrichtung zur Erfassung und/oder Erstellung von Etiketten von den oder für die Blutkonserven. Derartige Etiketten dienen der Identifizierung der jeweiligen Blutkonserven und können einen eindeutigen Code, insbesondere einen Barcode oder QR-Code, aufweisen. Bevorzugt ist die Etikettenvorrichtung in räumlicher Nähe zur Durchreichvorrichtung angeordnet. Die Erfassung der Etiketten kann neben der Erkennung von Barcodes oder QR-Codes auch eine OCR-basierte Texterkennung umfassen.

Zudem umfasst das Blutkonservendepot eine Steuereinrichtung zur Ansteuerung des Handhabungsroboters und der Etikettenvorrichtung.

Erfindungsgemäß ist der Handhabungsroboter eingerichtet, einzulagernde, in einem sich zumindest teilweise im Handhabungsbereich befindenden Einlagerungsbereich der Durchreichvorrichtung bereitzustellende Blutkonserven von dem Einlagerungsbereich zu entnehmen und in die Kühlvorrichtung einzulagern sowie auszulagernde Blutkonserven aus der Kühlvorrichtung zu entnehmen und in einem sich zumindest teilweise im Handhabungsbereich befindenden, zumindest teilweise von dem Einlagerungsbereich getrennten Auslagerungsbereich der Durchreichvorrichtung bereitzustellen. Mit anderen Worten ist die Handhabung der Blutkonserven, insbesondere deren Einlagerung und Auslagerung, mittels des Handhabungsroboters automatisiert durchführbar.

Durch die Erfindung wird eine kosteneffiziente und effiziente Handhabung von Blutkonserven ermöglicht.

Die Erfindung umfasst zudem Weiterbildungen, durch die sich weitere technische und/oder wirtschaftliche Vorteile ergeben.

Gemäß einer Ausgestaltung der Erfindung ist vorgesehen, dass die Durchreichvorrichtung eine Einlagerungsschublade und/oder eine Auslagerungsschublade umfasst. Die Einlagerungsschublade ist in eine Auszugposition versetzbar, in der einzulagernde Blutkonserven in die Einlagerungsschublade hineingelegt werden können. Die Einlagerungsschublade ist weiterhin in eine Einschubposition versetzbar, in der sich die Einlagerungsschublade zumindest teilweise im Einlagerungsbereich befindet. Die Auslagerungsschublade ist in eine Auszugposition versetzbar, in der auszulagernde Blutkonserven aus der Auslagerungsschublade entnommen werden können. Die Auslagerungsschublade ist zudem in eine Einschubposition versetzbar, in der sich die Auslagerungsschublade zumindest teilweise im Auslagerungsbereich befindet.

Hierdurch ergibt sich der Vorteil, dass für das Einlagern und Auslagern von Blutkonserven mittels der jeweiligen Schubladen jeweils zwei definierte Positionen vorgesehen sind. In der jeweiligen Auszugposition kann eine betreffende Blutkonserve zur Einlagerung manuell eingelegt werden, wenn sich die Einlagerungsschublade in ihrer Auszugposition befindet, beziehungsweise zur Auslagerung manuell entnommen werden, wenn sich die Auslagerungsschublade in ihrer Auszugposition befindet.

In der jeweiligen Einschubposition kann der Handhabungsroboter auf die betreffende Blutkonserve zugreifen, indem er diese aus der Einlagerungsschublade entnimmt, wenn sich die Einlagerungsschublade in ihrer Einschubposition befindet, beziehungsweise indem er diese in die Auslagerungsschublade ablegt, wenn sich die Auslagerungsschublade in ihrer Einschubposition befindet. In der jeweiligen Einschubposition der Schubladen befindet sich die betreffende Blutkonserve jeweils im Handhabungsbereich.

Es ist vorgesehen, dass sich die Einlagerungsschublade zumindest teilweise außerhalb des Handhabungsbereichs befindet, wenn sie sich in ihrer Auszugposition befindet, und/oder dass sich die Auslagerungsschublade zumindest teilweise außerhalb des Handhabungsbereichs befindet, wenn sie sich in ihrer Auszugposition befindet. Das Einlegen der betreffenden Blutkonserve in die Einlagerungsschublade und/oder das Entnehmen der betreffenden Blutkonserve aus der Auslagerungsschublade erfolgt vorzugsweise manuell, beispielsweise durch Bedienpersonal, insbesondere Laborpersonal.

Gemäß einer weiteren Ausgestaltung ist das Blutkonservendepot so ausgebildet, dass die Schutzeinhausung die Durchreichvorrichtung derart umschließt, dass ein Inneres der Einlagerungsschublade gegenüber der Umgebung abgeschirmt ist, wenn sich diese in ihrer Einschubposition befindet, und/oder ein Inneres der Auslagerungsschublade gegenüber der Umgebung abgeschirmt ist, wenn sich diese in ihrer Einschubposition befindet. Mit anderen Worten schirmt die Schutzeinhausung das Innere der Einlagerungsschublade und/oder das Innere der Auslagerungsschublade nach außen hin ab, wenn sich die jeweilige Schublade in der jeweiligen Einschubposition befindet. Hierdurch kann verhindert werden, dass Bedienpersonal Zugriff auf die jeweilige Schublade erhält, wenn sich diese in der jeweiligen Einschubposition befindet. Dadurch kann die Sicherheit bei der Bedienung des Blutkonservendepots erhöht werden, insbesondere wenn mit dem Handhabungsroboter Blutkonserven im jeweiligen Bereich der jeweiligen Schublade gehandhabt werden.

Die Durchreichvorrichtung kann ferner eine durch die Steuereinrichtung ansteuerbare Verriegelungseinrichtung umfassen, mittels der die Einlagerungsschublade in ihrer Einschubposition und/oder die Auslagerungsschublade in ihrer Einschubposition ortsfest verriegelbar sowie zum Versetzen in die jeweilige Auszugposition freigebbar ist. Hierdurch kann die Sicherheit bei der Bedienung des Blutkonservendepots weiter erhöht werden. Insbesondere kann mittels der Verriegelungseinrichtung verhindert werden, dass Bedienpersonal eine der Schubladen auszieht, also von der jeweiligen Einschubposition in die jeweilige Auszugposition versetzt, während mittels des Handhabungsroboters in Bezug auf die betreffende Schublade gerade eine Blutkonserve gehandhabt wird. Das Schalten der Verriegelungseinrichtung, also das Verriegeln beziehungsweise Freigeben der betreffenden Schublade in Bezug auf ihre jeweilige Position, erfolgt insbesondere durch eine entsprechende Ansteuerung mittels der Steuereinrichtung.

Vorzugsweise umfasst die Etikettenvorrichtung eine Etikettenscaneinrichtung zur Erfassung von den Blutkonserven zugeordneten Primäretiketten, Sekundäretiketten und/oder Entnahmebelegen. Ein Primäretikett ist ein Etikett, das in Bezug auf die betreffende Blutkonserve bereits vorhanden ist, wenn diese dem Blutkonservendepot, insbesondere über die Einlagerungsschublade, zugeführt wird. Ein jeweiliges Primäretikett wird beispielsweise von einem Labor erstellt, das die Blutkonserve zuvor handhabt, und als entsprechender Code auf der Blutkonserve aufgebracht. Vorzugsweise ist jeweils ein Primäretikett auf der Blutkonserve aufgebracht. Ein Sekundäretikett ist ein einer jeweiligen Blutkonserve zugeordnetes Etikett, das erst im Zusammenhang mit der Handhabung dieser Blutkonserve durch das Blutkonservendepot erstellt wird. Insbesondere ist vorgesehen, dass jeweils ein Sekundäretikett auf der Blutkonserve aufgebracht wird. Ein Entnahmebeleg ist ein Beleg, der im Zusammenhang mit der Auslagerung und Entnahme einer betreffenden Blutkonserve verwendet wird und Informationen zu der auszulagernden Blutkonserve umfasst. Insbesondere ist vorgesehen, dass unter Verwendung eines Entnahmebelegs eine aus dem Blutkonservendepot zu entnehmende Blutkonserve zur Auslagerung bereitgestellt wird. Hierbei ist vorgesehen, dass in Reaktion auf ein Erfassen eines solchen Entnahmebelegs mittels des Handhabungsroboters eine zur Auslagerung der betreffenden Blutkonserve erforderliche Handhabung durchgeführt wird.

Die Etikettenscaneinrichtung kann einen Primärscanner zur Erfassung von Primäretiketten von Blutkonserven umfassen, der auf einen zumindest teilweise von dem Einlagerungsbereich und dem Auslagerungsbereich getrennten Zwischenbereich der Durchreichvorrichtung gerichtet ist. Der Handhabungsroboter ist ferner dazu eingerichtet, einzulagernde Blutkonserven vor dem Einlagern in die Kühlvorrichtung und/oder auszulagernde Blutkonserven vor dem Bereitstellen im Auslagerungsbereich temporär in den Zwischenbereich zu bewegen, um die Erfassung des jeweiligen Primäretiketts zu ermöglichen. Hierdurch ergibt sich der Vorteil, dass die Erfassung eines jeweiligen Primäretiketts einer Blutkonserve automatisiert erfolgen kann, wodurch die Anzahl händisch durchzuführender Handhabungsschritte weiter reduziert wird. Das temporäre Bewegen einer einzulagernden Blutkonserve in den Zwischenbereich erfolgt nach deren Entnahme aus dem Einlagerungsbereich, während das temporäre Bewegen einer auszulagernden Blutkonserve in den Zwischenbereich nach deren Entnahme aus der Kühlvorrichtung erfolgt. Das temporäre Bewegen umfasst bevorzugt auch eine zeitweise Ablage der Blutkonserve im Zwischenbereich, wobei die Erfassung des jeweiligen Primäretiketts vorzugsweise erfolgt, wenn die Blutkonserve im Zwischenbereich abgelegt ist. Bevorzugt wird jeweils nur eine einzelne Blutkonserve temporär in den Zwischenbereich bewegt, sodass sich zu jedem Zeitpunkt jeweils eine einzelne Blutkonserve im Zwischenbereich befindet. Der Primärscanner ist vorzugsweise als Scan-Kamera ausgebildet; dies ermöglicht eine bildbasierte Erkennung des jeweiligen Primäretiketts. Der Zwischenbereich kann beispielsweise als definierte Zwischenablage ausgebildet sein, auf der eine einzelne Blutkonserve temporär abgelegt wird, wobei dem Zwischenbereich bevorzugt eine Kamera zur optischen Erfassung von Etiketten, insbesondere Barcodes, QR-Codes und/oder mittels OCR erfassbarer Informationen, zugeordnet ist.

Ferner ist vorgesehen, dass die Etikettenscaneinrichtung einen Sekundärscanner zur Erfassung von auszulagernden Blutkonserven zugeordneten Sekundäretiketten und/oder Entnahmebelegen umfasst. Der Sekundärscanner ist vorzugsweise als Handscanner ausgebildet; dies ermöglicht eine einfache Handhabung durch Bedienpersonal.

Gemäß einer weiteren Ausgestaltung ist die Etikettenscaneinrichtung dazu eingerichtet, Entnahmebelege zu erfassen, wobei die Steuereinrichtung dazu eingerichtet ist, als Reaktion auf das Erfassen eines einer auszulagernden Blutkonserve zugeordneten Entnahmebelegs diese Blutkonserve mittels des Handhabungsroboters im Auslagerungsbereich bereitzustellen. Hierdurch wird eine einfache, eindeutige und damit sichere Initiierung eines die jeweilige Blutkonserve betreffenden Entnahme- und Auslagerungsprozesses ermöglicht. Insbesondere ist vorgesehen, dass zur Entnahme einer Blutkonserve aus dem Blutkonservendepot die folgenden Schritte durchgeführt werden: Zunächst wird ein Entnahmebeleg mittels des Sekundärscanners erfasst. In Reaktion hierauf werden Informationen ermittelt, die die zu entnehmende Blutkonserve betreffen, sodass die betreffende Blutkonserve durch das Scannen des Entnahmebelegs angefordert wird. Daraufhin wird die anzufordernde Blutkonserve mittels des Handhabungsroboters im Auslagerungsbereich zur Entnahme durch das Bedienpersonal bereitgestellt. Anschließend wird ein der betreffenden Blutkonserve zugeordnetes Sekundäretikett auf die Blutkonserve aufgebracht, beispielsweise durch das Bedienpersonal oder durch den Handhabungsroboter. Danach wird das auf die Blutkonserve aufgebrachte Sekundäretikett mittels des Sekundärscanners erfasst, um eine Plausibilitätsprüfung durchzuführen. Diese Plausibilitätsprüfung kann einen Abgleich zwischen dem Entnahmebeleg und dem Sekundäretikett umfassen und/oder als Quittierung dienen, dass die betreffende Blutkonserve final entnommen wurde. In Reaktion auf eine solche Quittierung können weitere Handhabungsschritte mittels des Handhabungsroboters durchgeführt werden, beispielsweise das Auslagern weiterer Blutkonserven, insbesondere in entsprechender Weise wie bei der zuvor ausgelagerten Blutkonserve.

Vorzugsweise ist die Steuereinrichtung dazu eingerichtet, einen erfassten Entnahmebeleg einer auszulagernden Blutkonserve mit einem erfassten Primäretikett dieser Blutkonserve abzugleichen. Hierdurch wird überprüfbar, ob die durch den Entnahmebeleg angeforderte Blutkonserve tatsächlich derjenigen Blutkonserve entspricht, die im Zuge des Entnahmevorgangs mittels des Handhabungsroboters gehandhabt wird. Ergibt sich hierbei eine Abweichung, ist insbesondere vorgesehen, dass die betreffende Blutkonserve, die als abweichende Blutkonserve identifiziert wurde, zum Beispiel mittels des Handhabungsroboters wieder in die Kühlvorrichtung zurückverbracht wird. Der vorgenannte Abgleich erfolgt insbesondere im Zusammenhang mit der vorstehend beschriebenen Plausibilitätsprüfung.

Das Blutkonservendepot kann ferner eine durch die Steuereinrichtung ansteuerbare Datenschnittstelle umfassen, über die das Blutkonservendepot mit einem Labor-Informationssystem (LIS) kommuniziert. In dem Labor-Informationssystem sind digitale Daten gespeichert oder speicherbar, die Blutkonserven betreffen, die mit dem Blutkonservendepot in Zusammenhang stehen, das heißt digitale Daten zu den dem Blutkonservendepot zugeordneten Blutkonserven. Dies ermöglicht einen externen Zugriff auf entsprechende Daten. Beispielsweise kann abgefragt werden, ob eine bestimmte Blutkonserve oder eine Blutkonserve eines bestimmten Typs im Blutkonservendepot eingelagert ist. Mit dem Blutkonservendepot in Zusammenhang stehende Blutkonserven können insbesondere einzulagernde Blutkonserven, Blutkonserven während des Einlagerungsvorgangs, eingelagerte Blutkonserven, auszulagernde Blutkonserven, Blutkonserven während des Auslagerungsvorgangs sowie bereits ausgelagerte Blutkonserven umfassen.

Ferner ist vorzugsweise vorgesehen, dass die Steuereinrichtung dazu eingerichtet ist, als Reaktion auf das Erfassen eines einer auszulagernden Blutkonserve zugeordneten Entnahmebelegs, vor dem Bereitstellen dieser Blutkonserve mittels des Handhabungsroboters im Auslagerungsbereich, einen Abgleich dieses Entnahmebelegs mit die auszulagernde Blutkonserve betreffenden, im Labor-Informationssystem gespeicherten digitalen Daten durchzuführen. Mit anderen Worten wird die Plausibilitätsprüfung ferner unter Verwendung des Labor-Informationssystems durchgeführt. Hierdurch ergibt sich die Möglichkeit, die Plausibilitätsprüfung zentral und unter zusätzlicher Berücksichtigung von Daten durchzuführen, die nicht im Zuge der Einlagerung und/oder Auslagerung der betreffenden Blutkonserve erfasst wurden. Dadurch können Redundanzen bei der Datenspeicherung vermieden und die Effizienz von Prozessen im Zusammenhang mit dem Blutkonservendepot erhöht werden.

Vorzugsweise umfasst die Etikettenvorrichtung ferner einen Etikettendrucker zur Erstellung von Sekundäretiketten für auszulagernde Blutkonserven. Die Sekundäretiketten können hiermit unmittelbar vor Ort erstellt werden. Dadurch lassen sich Fehler bei der Zuordnung von Sekundäretiketten zu der jeweiligen Blutkonserve vermeiden, die andernfalls bei einem Transport der Sekundäretiketten von extern zu dem Blutkonservendepot auftreten könnten. Das gedruckte Sekundäretikett wird im Regelfall durch das Bedienpersonal auf die betreffende Blutkonserve aufgebracht.

Das Blutkonservendepot kann ferner einen in der Durchreichvorrichtung angeordneten und auf den Einlagerungsbereich gerichteten Abstandssensor umfassen, der dazu eingerichtet ist, einen Öffnungszustand eines Transportbehälters, in dem einzulagernde Blutkonserven im Einlagerungsbereich bereitgestellt sind, und/oder eine Stapelhöhe eines aus den einzulagernden Blutkonserven im Transportbehälter gebildeten Stapels zu erfassen. Hierdurch kann auf einfache Weise sichergestellt werden, ob und in welcher Weise mittels des Handhabungsroboters eine Entnahme einer jeweiligen Blutkonserve aus dem Einlagerungsbereich vorzunehmen ist. Ob eine Entnahme erfolgt, kann unter Berücksichtigung der durch den Abstandssensor erfassten Information darüber entschieden werden, ob im Einlagerungsbereich überhaupt noch eine Blutkonserve bereitgestellt ist. Wie die Entnahme erfolgt, kann unter Berücksichtigung der durch den Abstandssensor erfassten Stapelhöhe entschieden werden, insbesondere in welcher Höhe die jeweils, vorzugsweise als nächste, zu entnehmende Blutkonserve im Einlagerungsbereich angeordnet ist. Sind beispielsweise mehrere Blutkonserven im Einlagerungsbereich übereinander gestapelt, ist die als nächste zu entnehmende Blutkonserve höher angeordnet als bei einer geringeren Anzahl übereinander gestapelter Blutkonserven. Der Transportbehälter ist vorzugsweise zur Aufnahme einer begrenzten Anzahl von Blutkonserven ausgebildet, insbesondere zur Aufnahme von bis zu 20 Blutkonserven, die bevorzugt übereinander gestapelt bereitgestellt werden. Bevorzugt weist der Transportbehälter einen Deckel auf, sodass anhand der mittels des Abstandssensors erfassten Information ermittelbar ist, ob der Deckel auf dem Transportbehälter aufliegt (geschlossen) oder von diesem abgehoben (geöffnet) ist. Vorzugsweise dient die durch den Abstandssensor erfasste Information als Startbedingung für einen automatisierten Einlagerungsvorgang, insbesondere um zu erkennen, ob ein Transportbehälter im Einlagerungsbereich bereitgestellt ist und ob dessen Deckel geöffnet ist. Der Abstandssensor ist vorzugsweise ein laserbasierter Sensor.

Der Handhabungsroboter weist regelmäßig eine Greifeinrichtung (auch als Endeffektor bezeichnet) zum Greifen der handzuhabenden Blutkonserven auf. Die Greifeinrichtung ist dazu eingerichtet, die handzuhabenden Blutkonserven auf Basis von Unterdruck, Reibkraft und/oder Formschluss zu greifen, das heißt, temporär zu halten. Die Greifeinrichtung ist vorzugsweise durch die Steuereinrichtung ansteuerbar.

Die Greifeinrichtung kann zum Greifen handzuhabender Blutkonserven einen ersten Greifkopf sowie einen von dem ersten Greifkopf separat betätigbaren zweiten Greifkopf umfassen. Abhängig von der jeweiligen Greifanforderung kann hierdurch ein geeigneter Greifkopf eingesetzt werden. Der erste Greifkopf ist insbesondere als ein auf Unterdruck basierender Sauggreifer ausgebildet. Insbesondere bei flach liegenden Blutkonserven ergibt sich hierdurch der Vorteil, dass diese, regelmäßig nicht formstabilen, Blutkonserven sicher gegriffen werden können. Der zweite Greifkopf ist insbesondere als ein auf Reibkraft und/oder Formschluss basierender Parallelgreifer ausgebildet. Insbesondere bei stehend angeordneten oder stehend anzuordnenden Blutkonserven ergibt sich hierdurch der Vorteil, dass diese, insbesondere nicht formstabilen, Blutkonserven sicher gegriffen werden können. Die vorstehend beschriebene Ausgestaltung des ersten und des zweiten Greifkopfes ermöglicht, sowohl liegende als auch stehende Blutkonserven sicher zu greifen.

Die Schutzeinhausung ist vorzugsweise zumindest teilweise transparent ausgebildet. Hierdurch wird ermöglicht, dass von außen, insbesondere durch Bedienpersonal, die Handhabung von Blutkonserven mittels des Handhabungsroboters, beispielsweise stichprobenartig, überprüft werden kann. Insbesondere ist die Schutzeinhausung in einem oberen Bereich zumindest teilweise transparent ausgebildet. Beispielsweise kann die Schutzeinhausung in dem transparenten Bereich transparentes Polycarbonat umfassen oder aus diesem ausgebildet sein. In nicht transparenten Bereichen können Metallverbundplatten, insbesondere Aluminiumverbundplatten, als Komponenten der Schutzeinhausung eingesetzt sein.

Gemäß einer weiteren Ausgestaltung umfasst das Blutkonservendepot eine Sicherheitstechnik mit integrierter Sensorik zur Überwachung von Betriebszuständen und Handhabungsprozessen sowie zur Gewährleistung der Betriebssicherheit. Die Sicherheitstechnik umfasst eine Mehrzahl von an unterschiedlichen Komponenten des Blutkonservendepots angeordneten Sensoren, die Betriebs- und Zustandsinformationen erfassen. Die integrierte Sensorik kann insbesondere Positionssensoren zur Erfassung von Positionen des Handhabungsroboters und/oder der Greifeinrichtung, Näherungs- und/oder Lichtschrankensensoren zur Überwachung von Bewegungsräumen, Kraft- und/oder Drehmomentsensoren zur Erkennung von Kollisionen oder Blockaden, sowie Sensoren zur Überwachung von Öffnungs- und Schließzuständen von Türen und/oder Schubladen und/oder zur Erkennung eines Zugriffs von Bedienpersonal in sicherheitsrelevante Bereiche umfassen. Die Steuereinrichtung ist dazu eingerichtet, die Sensorsignale auszuwerten und in Abhängigkeit hiervon sicherheitsrelevante Maßnahmen einzuleiten, insbesondere ein Anhalten des Handhabungsroboters, ein Unterbrechen eines laufenden Handhabungsprozesses, ein Sperren von Bewegungen, ein Verriegeln der Durchreichvorrichtung und/oder das Auslösen einer Alarmierung. Auf diese Weise können Gefährdungen von Bedienpersonal sowie Beschädigungen von Blutkonserven und Systemkomponenten vermieden oder reduziert werden. Vorzugsweise ist die Sicherheitstechnik zur zyklischen Selbstüberwachung der Sensorik und/oder sicherheitsrelevanter Aktoren eingerichtet, wobei die Steuereinrichtung bei Erkennen einer Fehlfunktion den automatisierten Betrieb sperrt und einen sicheren Betriebszustand einnimmt. Insbesondere sind Türen der Schutzeinhausung jeweils mindestens ein Türsicherheitsschalter zugeordnet, der einen Öffnungszustand der Tür erfasst, wobei die Steuereinrichtung bei geöffneter Tür den Handhabungsroboter in einen sicheren Zustand versetzt.

Gemäß einer weiteren Ausgestaltung umfasst das Blutkonservendepot eine Temperaturüberwachungseinrichtung zur Überwachung der Temperatur zumindest innerhalb der Kühlvorrichtung. Die Temperaturüberwachungseinrichtung umfasst hierzu zumindest einen, bevorzugt mehrere, Temperatursensoren, die in der Kühlvorrichtung und/oder in deren Umgebung angeordnet sind. Die Steuereinrichtung ist dazu eingerichtet, erfasste Temperaturwerte zu speichern und zu protokollieren, um eine Temperaturhistorie für die gelagerten Blutkonserven bereitzustellen. Bei Über- und/oder Unterschreiten eines vorgegebenen Temperaturbereichs, insbesondere eines Bereichs von 2 °C bis 6 °C, wird bevorzugt eine Alarmierung ausgelöst, die lokal und/oder über eine Datenschnittstelle an ein externes System übertragbar ist. Zusätzlich oder alternativ kann die Steuereinrichtung bei einem Temperaturalarm eine Auslagerung betroffener Blutkonserven sperren und/oder diese als nicht freigegeben kennzeichnen, um eine weitere Verwendung zu verhindern.

Gemäß einer weiteren Ausgestaltung ist die Steuereinrichtung dazu eingerichtet, ein Ereignisprotokoll zu führen, in dem Handhabungsvorgänge im Zusammenhang mit Einlagerung, Umlagerung und/oder Auslagerung von Blutkonserven gespeichert werden. Das Ereignisprotokoll umfasst insbesondere Identifikationsdaten der Blutkonserve, einen Zeitstempel, eine Art des Vorgangs sowie optional eine Kennung eines zugehörigen Auftrags. Vorzugsweise wird zusätzlich eine Benutzerkennung erfasst, die einem Bediener zugeordnet ist, der den jeweiligen Vorgang initiiert oder bestätigt. Das Ereignisprotokoll wird bevorzugt manipulationsgeschützt gespeichert und kann zusätzlich oder alternativ über eine Datenschnittstelle an ein externes System, insbesondere ein Labor-Informationssystem, übertragen werden.

Gemäß einer weiteren Ausgestaltung ist das Blutkonservendepot hinsichtlich Kontaminationsschutz und Reinigbarkeit optimiert. Hierzu sind zumindest ausgewählte Oberflächen im Depotbereich und/oder in der Durchreichvorrichtung glattflächig, fugenarm und/oder desinfektionsmittelbeständig ausgebildet, sodass eine Reinigung und Desinfektion erleichtert wird.

Gemäß einer weiteren Ausgestaltung ist ein Notfallmodus vorgesehen, der einen manuellen Zugriff auf Blutkonserven ermöglicht, insbesondere bei Störungen des automatisierten Betriebs. Der Notfallmodus ist bevorzugt nur nach erfolgreicher Berechtigungsprüfung aktivierbar, wobei eine Authentifizierung eines Benutzers über geeignete Mittel erfolgt.

Im Notfallmodus wird der Handhabungsroboter bevorzugt in einen sicheren Zustand versetzt, und es kann eine kontrollierte Freigabe von Zugängen und/oder Schubladen erfolgen. Jeder Zugriff im Notfallmodus wird vorzugsweise im Ereignisprotokoll gespeichert. Eine Rückkehr in den automatisierten Betrieb erfolgt bevorzugt erst nach erneuter Berechtigungsprüfung und/oder nach Durchführung einer Plausibilitäts- oder Bestandsprüfung.

Ein zweiter Aspekt der Erfindung betrifft ein Verfahren zur Handhabung von Blutkonserven unter Verwendung eines vorstehend beschriebenen Blutkonservendepots. Die nachfolgend aufgeführten Verfahrensschritte werden jeweils durch Ansteuerung mittels der Steuereinrichtung bewirkt.
Dabei werden Etiketten für Blutkonserven mittels der Etikettenvorrichtung erfasst und/oder erstellt. Zudem werden im Einlagerungsbereich bereitgestellte Blutkonserven aus dem Einlagerungsbereich entnommen und in die Kühlvorrichtung eingelagert und/oder auszulagernde Blutkonserven aus der Kühlvorrichtung entnommen und im Auslagerungsbereich mittels des Handhabungsroboters bereitgestellt. Sämtliche vorstehend in Bezug auf das Blutkonservendepot und die damit in Zusammenhang stehenden Komponenten im Rahmen des ersten Aspekts der Erfindung beschriebenen Ausgestaltungen, Wirkungen und Vorteile gelten in entsprechender Weise auch für den zweiten Aspekt der Erfindung. Vor dem Entnehmen der Blutkonserven kann beispielsweise geprüft werden, ob ein Transportbehälter im Einlagerungsbereich bereitgestellt ist und ob ein Öffnungszustand des Transportbehälters einen Zugriff durch den Handhabungsroboter erlaubt. Die Anforderung einer auszulagernden Blutkonserve kann elektronisch erfolgen, insbesondere über ein Labor-Informationssystem, wobei im Zusammenhang mit der Auslagerung ein Ausgabeprotokoll erzeugt wird. Vorzugsweise erfolgt im Zuge des Auslagerungsvorgangs eine erneute Erfassung des Primäretiketts durch den Handhabungsroboter, um eine zusätzliche Gegenprüfung der Identität der auszulagernden Blutkonserve durchzuführen. Nach dem Bereitstellen einer Blutkonserve im Auslagerungsbereich kann die Steuereinrichtung eine Bereitstellungsinformation ausgeben, insbesondere in Form eines optischen oder akustischen Signals, und die Auslagerungsschublade zur Entnahme freigeben.

Hinsichtlich weiterer Ausführungen und Ausgestaltungen des vorgeschlagenen Blutkonservendepots wird auf die deutsche Gebrauchsmusteranmeldung mit der Anmeldenummer 20 2025 101 178.6 Bezug genommen, deren Inhalte hiermit in diese Patentanmeldung aufgenommen werden.

Die Erfindung umfasst ferner Kombinationen der Merkmale der vorstehend beschriebenen Ausführungsformen. Sie erstreckt sich somit auch auf Realisierungen, die jeweils eine Kombination von Merkmalen mehrerer der beschriebenen Ausführungsformen aufweisen, sofern diese Ausführungsformen nicht ausdrücklich als einander ausschließend beschrieben sind.

Die Erfindung ist nachfolgend anhand eines Ausführungsbeispiels und mit Bezug auf die schematischen Zeichnungen näher erläutert, wobei gleiche oder ähnliche Merkmale mit gleichen Bezugszeichen versehen sind; dazu zeigen:
- Fig. 1:: ein automatisiertes Blutkonservendepot in einer perspektivischen Ansicht,
- Fig. 2:: das in Fig. 1 dargestellte Blutkonservendepot in einer Draufsicht,
- Fig. 3:: das in den Fig. 1 und 2 dargestellte Blutkonservendepot in einer Seitenansicht und
- Fig. 4:: ein Detail des in den Fig. 1 bis 3 dargestellten Blutkonservendepots.

Bei den im Folgenden erläuterten Ausführungsbeispielen handelt es sich um bevorzugte Ausführungsformen der Erfindung. Die in den Ausführungsbeispielen beschriebenen Komponenten stellen jeweils einzelne, unabhängig voneinander zu betrachtende Merkmale der Erfindung dar, die die Erfindung auch jeweils unabhängig voneinander weiterbilden. Die Offenbarung soll daher auch andere als die explizit beschriebenen Kombinationen der Merkmale der Ausführungsformen umfassen. Darüber hinaus sind die beschriebenen Ausführungsformen auch durch weitere der vorstehend beschriebenen Merkmale der Erfindung ergänzbar.

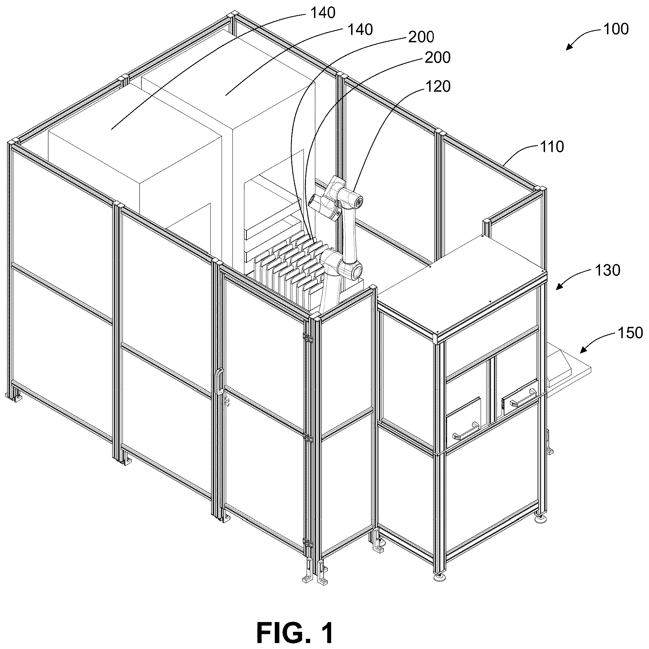

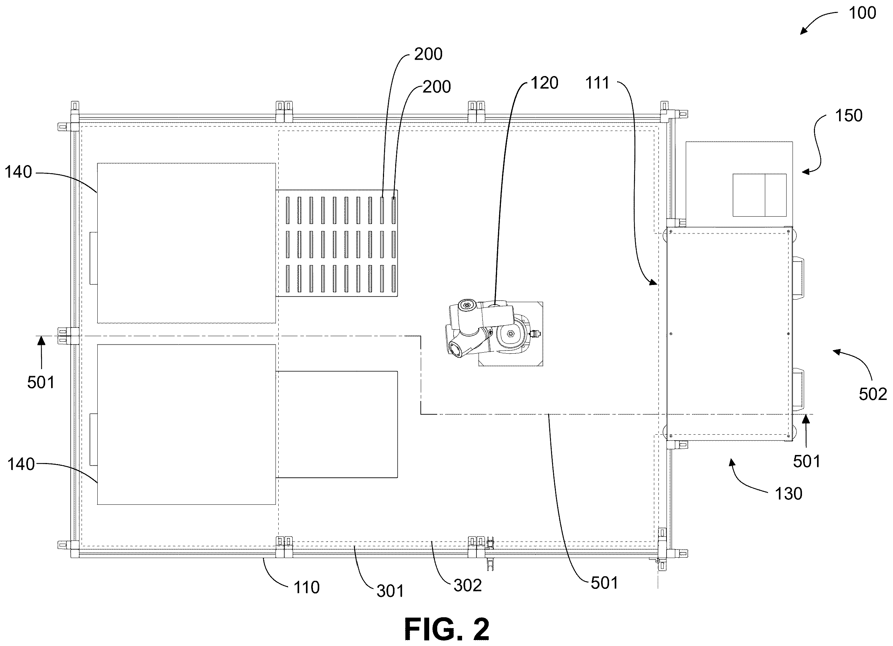

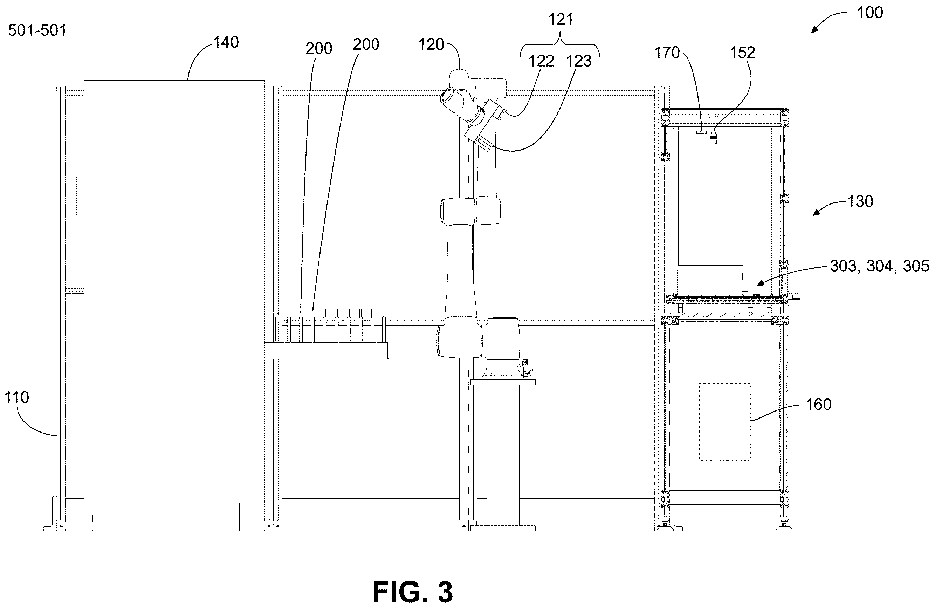

Das Blutkonservendepot 100 ist zur Handhabung von Blutkonserven 200 ausgebildet, von denen in den Fig. 1 bis 3 jeweils exemplarisch zwei mit dem Bezugszeichen 200 gekennzeichnet sind. Die in Fig. 3 dargestellte Seitenansicht ist eine Schnittansicht entlang der Schnittebene 501-501; die zugehörige gestufte Schnittebene 501 ist in Fig. 2 dargestellt.

Das Blutkonservendepot 100 umfasst die Schutzeinhausung 110, die den Depotbereich 301 zumindest teilweise gegenüber der Umgebung beziehungsweise nach außen hin abschirmt (siehe Fig. 2). Zudem umfasst das Blutkonservendepot 100 den Handhabungsroboter 120, der dazu eingerichtet ist, Blutkonserven 200 im Handhabungsbereich 302 zu handhaben. Rein exemplarisch umfasst der Handhabungsroboter 120 die Greifeinrichtung 121 zum Greifen der handzuhabenden Blutkonserven 200. Die Greifeinrichtung 121 weist exemplarisch den ersten Greifkopf 122 sowie den vom ersten Greifkopf 122 separat betätigbaren zweiten Greifkopf 123 auf. Hier ist der erste Greifkopf 122 beispielhaft als ein auf Unterdruck wirkender Sauggreifer ausgebildet, während der zweite Greifkopf 123 ein auf Reibkräften und/oder Formschluss wirkender Parallelgreifer ist.

Der Handhabungsbereich 302 befindet sich zumindest teilweise im Depotbereich 301 (siehe Fig. 2). Ferner umfasst das Blutkonservendepot 100 die Durchreichvorrichtung 130. Die Durchreichvorrichtung 130 ist an einer Durchreichöffnung 111 an den Depotbereich 301 angeschlossen. Die Durchreichöffnung 111 durchsetzt die Schutzeinhausung 110 (vgl. Fig. 2).

Das Blutkonservendepot 100 umfasst außerdem die im Depotbereich 301 angeordnete Kühlvorrichtung 140 zur Kühlung und Lagerung der Blutkonserven 200. Rein exemplarisch umfasst das Blutkonservendepot 100 zwei der Kühlvorrichtungen 140 (siehe Fig. 1 und 2).

Darüber hinaus umfasst das Blutkonservendepot 100 die Etikettenvorrichtung 150 zur Erfassung und/oder Erstellung von Etiketten von beziehungsweise für die Blutkonserven 200. Zudem umfasst das Blutkonservendepot 100 die Steuereinrichtung 160 zur Ansteuerung des Handhabungsroboters 120 und der Etikettenvorrichtung 150. Rein exemplarisch ist die Steuereinrichtung 160 in der Durchreichvorrichtung 130 angeordnet (siehe Fig. 3).

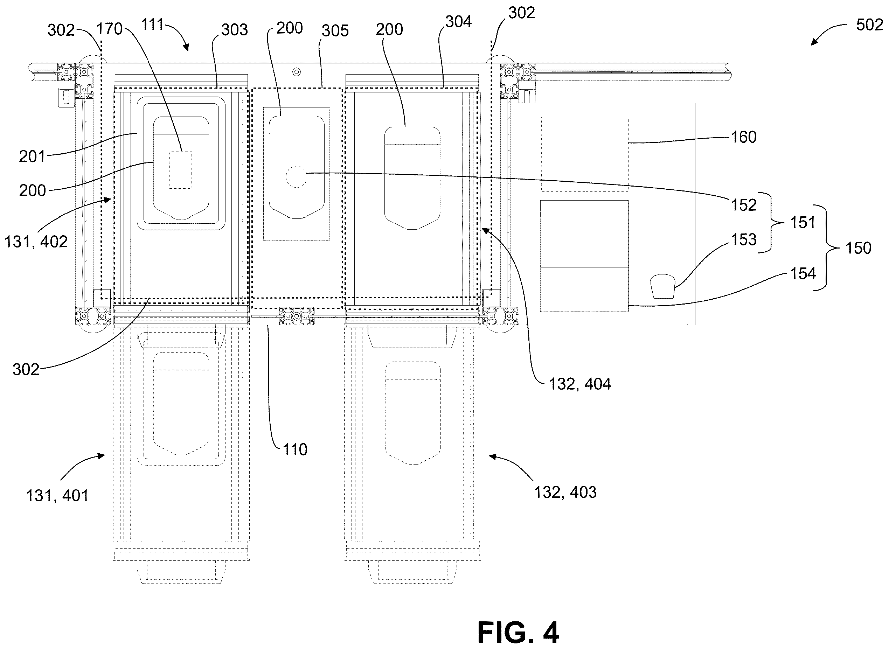

Fig. 4 zeigt ein Detail 502 des Blutkonservendepots 100. Die Position des Details 502 in Bezug auf das Blutkonservendepot 100 ist in Fig. 2 entsprechend mit dem Bezugszeichen 502 gekennzeichnet.

Der Handhabungsroboter 120 ist dazu eingerichtet, im Einlagerungsbereich 303 der Durchreichvorrichtung 130 bereitgestellte Blutkonserven 200 aus dem Einlagerungsbereich 303 zu entnehmen und in die Kühlvorrichtung 140 einzulagern. Ferner ist der Handhabungsroboter 120 dazu eingerichtet, auszulagernde Blutkonserven 200 aus der Kühlvorrichtung 140 zu entnehmen und im Auslagerungsbereich 304 der Durchreichvorrichtung 130 bereitzustellen. Der Auslagerungsbereich 304 und der Einlagerungsbereich 303 sind im Ausführungsbeispiel vollständig voneinander getrennt.

Die Durchreichvorrichtung 130 umfasst die Einlagerungsschublade 131 sowie die Auslagerungsschublade 132. Die Einlagerungsschublade 131 ist in die Auszugposition 401 versetzbar; in diese Auszugposition 401 können einzulagernde Blutkonserven 200 in die Einlagerungsschublade 131 eingelegt werden. Zudem ist die Einlagerungsschublade 131 in die Einschubposition 402 versetzbar; in ihrer Einschubposition 402 befindet sich die Einlagerungsschublade 131 im Einlagerungsbereich 303 und gleichzeitig im Handhabungsbereich 302. Die Auslagerungsschublade 132 ist in die Auszugposition 403 versetzbar; in ihrer Auszugposition 403 können auszulagernde Blutkonserven 200 aus der Auslagerungsschublade 132 entnommen werden. Zudem ist die Auslagerungsschublade 132 in die Einschubposition 404 versetzbar; in ihrer Einschubposition 404 befindet sich die Auslagerungsschublade 132 im Auslagerungsbereich 304 und gleichzeitig im Handhabungsbereich 302. In Fig. 4 befindet sich die Einlagerungsschublade 131 in ihrer Einschubposition 402 und die Auslagerungsschublade 132 ebenfalls in ihrer Einschubposition 404. Die Anordnung der Einlagerungsschublade 131 in ihrer Auszugposition 401 sowie die Anordnung der Auslagerungsschublade 132 in ihrer Auszugposition 403 sind in Fig. 4 jeweils mit gestrichelten Linien dargestellt.

Die Etikettenvorrichtung 150 umfasst ferner die Etikettenscaneinrichtung 151 zur Erfassung von den Blutkonserven 200 zugeordneten Primäretiketten, Sekundäretiketten und/oder Entnahmebelegen. Die Etikettenscaneinrichtung 151 umfasst den auf den Zwischenbereich 305 der Durchreichvorrichtung 130 gerichteten Primärscanner 152 zur Erfassung der Primäretiketten der Blutkonserven 200. Der Zwischenbereich 305 ist zumindest teilweise von dem Einlagerungsbereich 303 und dem Auslagerungsbereich 304 getrennt.

### Der Handhabungsroboter 120 ist dazu eingerichtet, einzulagernde

Blutkonserven 200 vor dem Einlagern in die Kühlvorrichtung 140 und/oder auszulagernde Blutkonserven 200 vor dem Bereitstellen im Auslagerungsbereich 304 temporär in den Zwischenbereich 305 zu bewegen, um die Erfassung des jeweiligen Primäretiketts zu ermöglichen. Ferner umfasst die Etikettenscaneinrichtung 151 den Sekundärscanner 153 zur Erfassung von den auszulagernden Blutkonserven 200 zugeordneten Sekundäretiketten und/oder Entnahmebelegen. Der Sekundärscanner 153 ist als Handscanner ausgebildet. Die Etikettenvorrichtung 150 umfasst ferner den Etikettendrucker 154 zur Erstellung von Sekundäretiketten für auszulagernde Blutkonserven 200.

Das Blutkonservendepot 100 weist ferner den Abstandssensor 170 auf. Der Abstandssensor 170 ist in der Durchreichvorrichtung 130 angeordnet und auf den Einlagerungsbereich 303 gerichtet. Der Abstandssensor 170 ist dazu eingerichtet, den Öffnungszustand des Transportbehälters 201, in dem einzulagernde Blutkonserven 200 im Einlagerungsbereich 303 bereitgestellt sind, und/oder die Stapelhöhe eines aus den einzulagernden Blutkonserven 200 im Transportbehälter 201 gebildeten Stapels zu erfassen.

Die Schutzeinhausung 110 umschließt die Durchreichvorrichtung 130 derart, dass das Innere der Einlagerungsschublade 131 gegenüber der Umgebung abgeschirmt ist, wenn sich diese in ihrer Einschubposition 402 befindet, und dass das Innere der Auslagerungsschublade 132 gegenüber der Umgebung abgeschirmt ist, wenn sich diese in ihrer Einschubposition 404 befindet.

Der vorstehend erläuterte Umstand, dass die Durchreichöffnung 111 die Schutzeinhausung 110 durchsetzt (siehe Fig. 2), steht hierzu nicht im Widerspruch. Das Durchsetzen der Schutzeinhausung 110 durch die Durchreichöffnung 111 bezieht sich vielmehr darauf, dass die Durchreichöffnung 111 an einer Außengrenze des Depotbereichs 301 angeordnet ist und der die Abschirmung des Depotbereichs 301 gegenüber der Umgebung bildende Teil der Schutzeinhausung 110 durch die Durchreichöffnung 111 unterbrochen ist. Der Teil der Schutzeinhausung 110, der exemplarisch die Durchreichvorrichtung 130 umschließt, ist hiervon nicht betroffen und umschließt die Durchreichvorrichtung 130 einschließlich der Durchreichöffnung 111.

### Bezugszeichenliste

- 100: Blutkonservendepot
- 110: Schutzeinhausung
- 111: Durchreichöffnung
- 120: Handhabungsroboter
- 121: Greifeinrichtung
- 122: erster Greifkopf
- 123: zweiter Greifkopf
- 130: Durchreichvorrichtung
- 131: Einlagerungsschublade
- 132: Auslagerungsschublade
- 140: Kühlvorrichtung
- 150: Etikettenvorrichtung
- 151: Etikettenscaneinrichtung
- 152: Primärscanner
- 153: Sekundärscanner
- 154: Etikettendrucker
- 160: Steuereinrichtung
- 170: Abstandssensor
- 200: Blutkonserve
- 201: Transportbehälter
- 301: Depotbereich
- 302: Handhabungsbereich
- 303: Einlagerungsbereich
- 304: Auslagerungsbereich
- 305: Zwischenbereich
- 401: Auszugposition der Einlagerungsschublade
- 402: Einschubposition der Einlagerungsschublade
- 403: Auszugposition der Auslagerungsschublade
- 404: Einschubposition der Auslagerungsschublade
- 501: Schnittebene
- 502: Detail

## Patentansprüche

1. Automatisiertes Blutkonservendepot (100) zur Handhabung von Blutkonserven (200), mit:
- einer Schutzeinhausung (110), die einen Depotbereich (301) zumindest teilweise gegenüber der Umgebung abschirmt,
- einem Handhabungsroboter (120), der dazu eingerichtet ist, Blutkonserven (200) in einem sich zumindest teilweise im Depotbereich (301) befindenden Handhabungsbereich (302) zu handhaben,
- einer Durchreichvorrichtung (130), die an einer die Schutzeinhausung (110) durchsetzenden Durchreichöffnung (111) an den Depotbereich (301) angeschlossen ist,
- mindestens einer im Depotbereich (301) angeordneten Kühlvorrichtung (140) zur Kühlung und Lagerung der Blutkonserven (200),
- einer Etikettenvorrichtung (150) zur Erfassung und/oder Erstellung von Etiketten von und/oder für die Blutkonserven (200), sowie
- einer Steuereinrichtung (160) zur Ansteuerung des Handhabungsroboters (120) und der Etikettenvorrichtung (150),
**dadurch gekennzeichnet, dass** der Handhabungsroboter (120) ferner dazu eingerichtet ist,
- einzulagernde, in einem sich zumindest teilweise im Handhabungsbereich (302) befindenden Einlagerungsbereich (303) der Durchreichvorrichtung (130) bereitgestellte Blutkonserven (200) aus dem Einlagerungsbereich (303) zu entnehmen und in die Kühlvorrichtung (140) einzulagern, sowie
- auszulagernde Blutkonserven (200) aus der Kühlvorrichtung (140) zu entnehmen und in einem sich zumindest teilweise im Handhabungsbereich (302) befindenden, zumindest teilweise von dem Einlagerungsbereich (303) getrennten Auslagerungsbereich (304) der Durchreichvorrichtung (130) bereitzustellen.

2. Blutkonservendepot (100) nach Anspruch 1, **dadurch gekennzeichnet, dass** die Durchreichvorrichtung (130) eine Einlagerungsschublade (131) und/oder eine Auslagerungsschublade (132) umfasst, wobei die Einlagerungsschublade (131) in eine Auszugposition (401) zum Hineinlegen einzulagernder Blutkonserven (200) in die Einlagerungsschublade (131) versetzbar ist, und wobei die
Einlagerungsschublade (131) in eine Einschubposition (402) versetzbar ist, in der sich die Einlagerungsschublade (131) zumindest teilweise im
Einlagerungsbereich (303) befindet, wobei die Auslagerungsschublade (132) in eine Auszugposition (403) zum Entnehmen auszulagernder Blutkonserven (200) aus der Auslagerungsschublade (132) versetzbar ist, und wobei die Auslagerungsschublade (132) in ihrer Einschubposition (404) versetzbar ist, in der sich die Auslagerungsschublade (132) zumindest teilweise im
Auslagerungsbereich (304) befindet.

3. Blutkonservendepot (100) nach Anspruch 2, **dadurch gekennzeichnet, dass** die Schutzeinhausung (110) die Durchreichvorrichtung (130) derart umschließt, dass ein Inneres der Einlagerungsschublade (131) gegenüber der Umgebung abgeschirmt ist, wenn sich diese in ihrer Einschubposition (402) befindet und/oder ein Inneres der Auslagerungsschublade (132) gegenüber der Umgebung abgeschirmt ist, wenn sich diese in ihrer Einschubposition (404) befindet.

4. Blutkonservendepot (100) nach Anspruch 2 oder 3, **dadurch gekennzeichnet, dass** die Durchreichvorrichtung (130) eine durch die Steuereinrichtung (160) ansteuerbare Verriegelungseinrichtung umfasst, mittels der die Einlagerungsschublade (131) in ihrer Einschubposition (402) und/oder die Auslagerungsschublade (132) in ihrer Einschubposition (404) ortsfest verriegelbar und zum Versetzen der Einlagerungsschublade (131) in ihrer Auszugposition (401) und/oder der Auslagerungsschublade (132) in ihrer Auszugposition (403) freigebbar ist.

5. Blutkonservendepot (100) nach einem der Ansprüche 1 bis 4, **dadurch gekennzeichnet, dass** die Etikettenvorrichtung (150) eine Etikettenscaneinrichtung (151) zur Erfassung von den Blutkonserven (200) zugeordneten Primäretiketten, Sekundäretiketten und/oder Entnahmebelegen umfasst.

6. Blutkonservendepot (100) nach Anspruch 5, **dadurch gekennzeichnet, dass** die Etikettenscaneinrichtung (151) einen auf einen zumindest teilweise von dem Einlagerungsbereich (303) und dem Auslagerungsbereich (304) getrennten Zwischenbereich (305) der Durchreichvorrichtung (130) gerichteten Primärscanner (152) zur Erfassung von Primäretiketten der Blutkonserven (200) umfasst, wobei der Handhabungsroboter (120) ferner dazu eingerichtet ist, einzulagernde Blutkonserven (200) vor dem Einlagern in die Kühlvorrichtung (140) und/oder auszulagernde Blutkonserven (200) vor dem Bereitstellen im Auslagerungsbereich (304) temporär in den Zwischenbereich (305) zur Erfassung von deren jeweiligem Primäretikett zu bewegen.

7. Blutkonservendepot (100) nach einem der Ansprüche 1 bis 6, **dadurch gekennzeichnet, dass** die Etikettenscaneinrichtung (151) einen Sekundärscanner (153) zur Erfassung von auszulagernden Blutkonserven (200) zugeordneten Sekundäretiketten und/oder Entnahmebelegen umfasst.

8. Blutkonservendepot (100) nach Anspruch 7, **dadurch gekennzeichnet, dass** die Etikettenscaneinrichtung (151) dazu eingerichtet ist, Entnahmebelege zu erfassen, wobei die Steuereinrichtung (160) dazu eingerichtet ist, als Reaktion auf das Erfassen eines einer auszulagernden Blutkonserve (200) zugeordneten Entnahmebelegs die betreffende Blutkonserve (200) mittels des Handhabungsroboters (120) im Auslagerungsbereich (304) bereitzustellen.

9. Blutkonservendepot (100) nach Anspruch 6 und 8, **dadurch gekennzeichnet, dass** die Steuereinrichtung (160) dazu eingerichtet ist, einen erfassten Entnahmebeleg einer auszulagernden Blutkonserve (200) mit einem erfassten Primäretikett dieser Blutkonserve (200) abzugleichen.

10. Blutkonservendepot (100) nach einem der Ansprüche 1 bis 9, **dadurch gekennzeichnet, dass** das Blutkonservendepot (100) ferner eine mittels der Steuereinrichtung (160) ansteuerbare Datenschnittstelle zur Kommunikation des Blutkonservendepots (100) mit einem Labor-Informationssystem umfasst, wobei im Labor-Informationssystem digitale Daten zu den dem Blutkonservendepot (100) zugeordneten Blutkonserven (200) gespeichert oder speicherbar sind.

11. Blutkonservendepot (100) nach Anspruch 8 oder 9 und Anspruch 10, **dadurch gekennzeichnet, dass** die Steuereinrichtung (160) dazu eingerichtet ist, als Reaktion auf das Erfassen eines einer auszulagernden Blutkonserve (200) zugeordneten Entnahmebelegs vor dem Bereitstellen dieser Blutkonserve (200) mittels des Handhabungsroboters (120) im Auslagerungsbereich (304) diesen Entnahmebeleg mit der auszulagernden Blutkonserve (200) zugeordneten, im Labor-Informationssystem gespeicherten digitalen Daten abzugleichen.

12. Blutkonservendepot (100) nach einem der Ansprüche 1 bis 11, **dadurch gekennzeichnet, dass** die Etikettenvorrichtung (150) einen Etikettendrucker (154) zur Erstellung von Sekundäretiketten für auszulagernde Blutkonserven (200) umfasst.

13. Blutkonservendepot (100) nach einem der Ansprüche 1 bis 12, **dadurch gekennzeichnet, dass** das Blutkonservendepot (100) einen in der Durchreichvorrichtung (130) angeordneten, auf den Einlagerungsbereich (303) gerichteten Abstandssensor (170) umfasst, der dazu eingerichtet ist, einen Öffnungszustand eines Transportbehälters (201) zur Bereitstellung einzulagernder Blutkonserven (200) im Einlagerungsbereich (303) und/oder eine Stapelhöhe eines aus den einzulagernden Blutkonserven (200) im Transportbehälter (201) gebildeten Stapels zu erfassen.

14. Blutkonservendepot (100) nach einem der Ansprüche 1 bis 13, **dadurch gekennzeichnet, dass** der Handhabungsroboter (120) eine Greifeinrichtung (121) zum Greifen der Blutkonserven (200) umfasst, die dazu eingerichtet ist, die Blutkonserven (200) auf Basis von Unterdruck, Reibkraft und/oder Formschluss zu greifen.

15. Verfahren zur Handhabung von Blutkonserven (200) unter Verwendung eines Blutkonservendepots (100) nach einem der Ansprüche 1 bis 14 mit den jeweils durch Ansteuerung mittels der Steuereinrichtung (160) bewirkten Schritten:
- Erfassen und/oder Erstellen von Etiketten von oder für Blutkonserven (200) mittels der Etikettenvorrichtung (150), und
- Entnehmen von im Einlagerungsbereich (303) bereitgestellten Blutkonserven (200) von dem Einlagerungsbereich (303) und Einlagern in die Kühlvorrichtung (140) mittels des Handhabungsroboters (120) und/oder Entnehmen auszulagernder Blutkonserven (200) aus der Kühlvorrichtung (140) und Bereitstellen im Auslagerungsbereich (304) mittels des Handhabungsroboters (120).
